# FASCICULE DE BREVET EUROPEEN

(11) **EP 3 897 633 B1**
(45) Date de publication et mention de la délivrance du brevet: **07.06.2023**
(21) Numéro de dépôt: 19827703.0
(22) Date de dépôt: 18.12.2019
(51) Int. Cl.: A61K 31/443, A61P 1/00

(54) **UTILISATION D'UN ANTAGONISTE DE PAR-1 POUR LE TRAITEMENT D'UNE MALADIE INFLAMMATOIRE CHRONIQUE INTESTINALE**
VERWENDUNG EINES PAR-1-ANTAGONISTEN ZUR BEHANDLUNG EINER CHRONISCHEN ENTZÜNDLICHEN DARMERKRANKUNG
USE OF A PAR-1 ANTAGONIST FOR THE TREATMENT OF A CHRONIC INFLAMMATORY INTESTINAL DISEASE

(30) Priorité: 19.12.2018 FR 1873359
(43) Date de publication de la demande: 27.10.2021
(73) Titulaire: Cvasthera, 81290 Labruguière (FR)
(72) Inventeur: LE GRAND, Bruno, 81220 Teyssode (FR); SABLAYROLLES, Sylvie, 81220 Teyssode (FR)
(74) Mandataire: Ipside
(86) Numéro de dépôt international: PCT/EP2019/086027
(87) Numéro de publication internationale: WO 2020/127539

(56) Documents cités:
- WO-A1-2010/136137
- WO-A1-2015/090705
- VERGNOLLE NATHALIE ET AL: "A role for proteinase-activated receptor-1 in inflammatory bowel diseases", JOURNAL OF CLINICAL INVESTIGATION, vol. 114, no. 10, novembre 2004 (2004-11), pages 1444-1456, XP002794003, ISSN: 0021-9738
- HOULE S., VERGNOLLE N.: "Récepteurs activés par des protéases et pathologies du tractus digestif", HÉPATO-GASTRO, vol. 13, no. 1, janvier 2006 (2006-01), pages 69-75, XP009515815,
- GLORO ROMAIN ET AL: "Increased expression of human protease-activated receptor 1 and 3 (HPAR-1 and -3) mRNA in inflammatory bowel disease (IBD) patients colonic mucosa", GASTROENTEROLOGY, vol. 132, no. 4, Suppl. 2, avril 2007 (2007-04), page A240, XP002794005, & DIGESTIVE DISEASE WEEK MEETING/108TH ANNUAL MEETING OF THE AMERICAN-GASTROENTEROLOGICAL-ASSOCIATION; WASHINGTON, DC, USA; MAY 19 -24, 2007 ISSN: 0016-5085
- DATABASE BIOSIS [Online] BIOSCIENCES INFORMATION SERVICE, PHILADELPHIA, PA, US; November 2004 (2004-11), VERGNOLLE NATHALIE ET AL: "A role for proteinase-activated receptor-1 in inflammatory bowel diseases", Database accession no. PREV200500068853 & VERGNOLLE NATHALIE ET AL: "A role for proteinase-activated receptor-1 in inflammatory bowel diseases", JOURNAL OF CLINICAL INVESTIGATION, vol. 114, no. 10, November 2004 (2004-11), pages 1444-1456, ISSN: 0021-9738
- DATABASE BIOSIS [Online] BIOSCIENCES INFORMATION SERVICE, PHILADELPHIA, PA, US; April 2007 (2007-04), GLORO ROMAIN ET AL: "Increased expression of human protease-activated receptor 1 and 3 (HPAR-1 and -3) mRNA in inflammatory bowel disease (IBD) patients colonic mucosa", Database accession no. PREV200700603599 & GASTROENTEROLOGY, vol. 132, no. 4, Suppl. 2, April 2007 (2007-04), page A240, DIGESTIVE DISEASE WEEK MEETING/108TH ANNUAL MEETING OF THE AMERICAN-GASTROENTEROLOGICAL-ASSOCIATION; WASHINGTON, DC, USA; MAY 19 -24, 2007 ISSN: 0016-5085

## Description

La présente invention s'inscrit dans le domaine du traitement des maladies chroniques intestinales.

Plus particulièrement, la présente invention concerne un antagoniste de PAR-1, et une composition pharmaceutique contenant un tel antagoniste, pour leur utilisation pour la prévention et/ou le traitement d'une maladie inflammatoire chronique de l'intestin et du côlon, en particulier pour réduire la douleur et/ou réparer les tissus épithéliaux de l'intestin chez un sujet atteint d'une telle maladie.

Les maladies inflammatoires chroniques de l'intestin et du côlon, également nommées maladies inflammatoires intestinales, couramment désignées par l'abréviation MICIs, comprennent la maladie de Crohn et la rectocolite hémorragique.

La maladie de Crohn peut atteindre la totalité du tube digestif, soit en sections contiguës ou soit en section isolée, mais elle touche en priorité l'intestin grêle et le colon. L'inflammation peut toucher la muqueuse interne et même traverser l'épaisseur entière de la paroi intestinale, elle se manifeste par de l'oedème, une dilatation des vaisseaux sanguins et une perte de fluide dans les tissus. La maladie de Crohn est une pathologie de l'adulte jeune qui débute généralement entre 20 et 30 ans. Il existe un second pic de fréquence entre 50 et 80 ans et 15% des cas concernent les enfants. Les deux sexes sont également atteints. L'affection est ubiquitaire mais son incidence est plus élevée au Nord qu'au Sud de l'Europe. En France, l'incidence de la maladie de Crohn est de 5 à 6 cas pour 100 000 habitants et autant de rectocolites hémorragiques. La prévalence augmente en revanche de façon exponentielle dans les pays en cours d'industrialisation, tels que les pays du Maghreb, d'Asie, d'Afrique du Sud...

L'origine de la maladie de Crohn est encore inconnue. Elle est probablement multifactorielle associant un terrain génétique prédisposé - il a été récemment mis en évidence chez l'homme sur les chromosomes 12 et 16, un gène de prédisposition aux maladies inflammatoires chroniques intestinales - et des facteurs environnementaux qui restent à élucider, par exemple une infection déclenchante ou encore la pollution. Par ailleurs, le rôle néfaste du tabac est clairement établi, augmentant le risque de récidive.

La rectocolite hémorragique, ou colite ulcéreuse, est une maladie inflammatoire chronique intestinale qui affecte l'extrémité distale du tube digestif, c'est-à-dire le côlon et le rectum qui est toujours touché. Son étiologie est inconnue, bien qu'une composante génétique constitue une hypothèse. Elle est classée dans les maladies auto-immunes. Cette maladie ne se guérit pas, ce qui nécessite un traitement médicamenteux à vie. L'objectif des traitements est que les rémissions durent le plus longtemps possible. Son diagnostic repose essentiellement sur les examens cytologiques qui accompagnent les prélèvements lors d'une coloscopie.

Ces maladies inflammatoires chroniques de l'intestin et du côlon évoluent par poussées inflammatoires de durée et de fréquence extrêmement variables selon les patients. Ces poussées alternent avec des phases de rémission.

Lors des poussées inflammatoires, les MICls se caractérisent le plus souvent par des douleurs abdominales, des diarrhées fréquentes, parfois sanglantes, ou encore une atteinte de la région anale (fissure, abcès). Ces symptômes font peser sur la maladie un certain tabou. Ils s'accompagnent souvent de fatigue, d'anorexie et de fièvre, voire de manifestations extra-intestinales (articulaires, cutanées, oculaires, hépatiques). Chez environ 20% des patients, les crises sont sévères : leur intensité peut imposer l'hospitalisation, l'arrêt de l'alimentation et un traitement par perfusion pendant quelques jours. En outre, l'évolution de la maladie peut entrainer le rétrécissement du segment intestinal atteint, puis une occlusion ou encore un abcès. Celui-ci peut conduire à la formation d'une fistule, c'est-à-dire l'ouverture d'une voie de communication anormale partant de l'intestin vers un autre organe. Ces complications nécessitent une intervention chirurgicale. Les MICls sont associées à un risque accru de cancer colorectal, notamment lorsque des lésions sont présentes au niveau du côlon.

Le diagnostic des MICls repose sur plusieurs critères cliniques, biologiques et d'imagerie médicale. Lorsque des symptômes cliniques évoquent une MICI, un bilan biologique est réalisé. Il permet de détecter un syndrome inflammatoire, la présence de marqueurs spécifiques des MICIs, notamment les anticorps *anti-Saccharomyces Cerevisiae* (ASCA) et les anticorps anti-cytoplasme des polynucléaires neutrophiles (ANCA). Une endoscopie digestive permet de rechercher la présence et la localisation de lésions du tube digestif, ainsi que de réaliser des prélèvements.

La prise en charge d'un patient atteint de MICI fait intervenir de très nombreux paramètres liés à la forme de la maladie et au patient lui-même. Cinq catégories de médicaments sont actuellement utilisées dans le traitement de base des MICIs. Il s'agit des salicylés, des corticoïdes, des immunosuppresseurs, des biothérapies et des antibiotiques.

Parmi les salicylés, le chef de file et le plus ancien est représenté par la sulfasalazine. Le risque d'effets indésirables associés à ce médicament a conduit à rechercher des dérivés mieux tolérés. Il s'agit principalement de la mésalazine et de l'olsalazine.

Point de passage quasi obligatoire à un moment ou à un autre de l'évolution de la maladie, la corticothérapie a mauvaise réputation. Elle fait craindre la survenue de nombreux effets gênants. Le traitement est habituellement débuté à doses élevées, puis les posologies sont progressivement diminuées. Dans tous les cas, les corticoïdes nécessitent une surveillance médicale.

Les médicaments qui diminuent les réactions du système immunitaire, ou immunosuppresseurs, sont de plus en plus utilisés pour le traitement des MICIs. Ils sont la base du traitement d'entretien de la maladie de Crohn, plus rarement du traitement d'entretien de la rectocolite. Ils ont un délai d'action de quelques mois. L'utilisation d'immunosuppresseurs impose une surveillance régulière de certains paramètres sanguins.

Les biothérapies consistent en l'emploi thérapeutique de produits dérivés des substances présentes dans l'organisme vivant. Le premier médicament de cette classe utilisé pour le traitement des MICls a été l'infliximab, c'est le seul produit autorisé actuellement par les autorités sanitaires. Il s'agit d'un anticorps chimérique dirigé contre le TNF-alpha. Il est administré par perfusion courte. Les effets secondaires sont dominés par les infections et les réactions d'hypersensibilité.

Enfin, les antibiotiques sont un traitement d'appoint des MICIs. Deux d'entre eux occupent une place particulière dans la prise en charge des localisations ano-rectales de la maladie de Crohn : ce sont le métronidazole et la ciprofloxacine. Ces deux composés ne sont cependant pas dénués d'effets secondaires.

Schématiquement, on distingue le traitement de la poussée visant à mettre le plus rapidement possible le tube digestif au repos, et le traitement d'entretien visant à maintenir le plus longtemps possible cette rémission.

A l'heure actuelle, il n'existe pas de traitement curatif des MICls. Les médicaments anti-inflammatoires actuels permettent souvent un contrôle durable de la maladie, même s'ils ne sont pas parfaits. Ils préviennent l'apparition des poussées et prolongent les phases de rémission en favorisant la cicatrisation des lésions du tube digestif.

En l'absence de remède efficace, les patients atteints de la maladie de Crohn nécessitent des soins médicaux continus.

En outre, la surface d'absorption de l'épithélium intestinal laisse une immense possibilité de passage dans le sang et dans la lymphe de molécules étrangères et toxiques. La rupture de cette barrière mécanique et le passage dans la circulation générale de ces substances, hyperperméabilité épithéliale, sont le point de départ commun à de multiples pathologies, et en particulier des maladies inflammatoires chroniques telles que la maladie de Crohn et la rectocolite hémorragique. Il n'existe pas de traitement actuel qui protège de cette translocation de la barrière épithéliale et/ou qui accélère le processus de cicatrisation des épithéliums lésés, les traitements actuels des maladies inflammatoires chroniques de l'intestin ayant comme objectif de réduire la poussée inflammatoire sans en réparer le substrat.

La présente invention vise à proposer de nouveaux composés permettant de lutter efficacement contre les MICIs.

La publication de Vergnolle et al., 2004, Journal of Clinical Investigation, 114(10): 1444-1456 décrit le rôle du récepteur PAR-1 dans les maladies inflammatoires de l'intestin. Ce document indique qu'un antagoniste particulier de PAR-1, le L-arininamide-4-méthoxy-N-[[[1-[92,6-dichlorophényl-méthyl]-3-(1-pyrrolidinylméthyl)-1H-indol-6-yl]amino]carbonyl]-1-phénylalanyl-N-(phénylméthyl)-(5), permet d'améliorer le taux de survie des animaux dans un modèle de colite induite chez la souris, en réduisant le phénomène d'inflammation.

Il a maintenant été découvert par les présents inventeurs, de manière tout à fait surprenante, que des antagonistes du récepteur PAR-1 particuliers permettent non seulement de diminuer le phénomène inflammatoire impliqué dans les maladies inflammatoires chroniques de l'intestin et du côlon, et notamment la maladie de Crohn, mais qu'ils permettent également de réduire la douleur qui y est associée ainsi que de réparer les tissus épithéliaux de l'intestin.

Le récepteur PAR-1 (pour l'anglais protease-activated receptor-1) est un récepteur heptahélicoïdal, couplé aux protéines G trimériques, composé de 425 acides aminés. La thrombine active le récepteur PAR-1 en clivant son extrémité N-terminale extracellulaire entre l'arginine 41 et la sérine 42. Le peptide clivé n'a pas d'activité particulière, la nouvelle extrémité N-terminale du récepteur joue le rôle d'agoniste en se repliant vers la surface cellulaire et en interagissant avec les domaines extracellulaires.

Le PAR-1 joue un rôle clé dans l'activation plaquettaire aux faibles concentrations de thrombine, si bien que son rôle a été établi dans le domaine de la biologie vasculaire et de l'athérothrombose. Des antagonistes PAR-1 ont émergé comme de nouveaux antithrombotiques prometteurs et actifs par voie orale. On peut citer à ce titre le vorapaxar et l'atopaxar qui ont fourni des données cliniques prometteuses (Capodanno et al., 2012, J. Thromb. Haemost. 10(10): 2006-15). Le vorapaxar a d'ailleurs obtenu l'enregistrement de la FDA en 2014 dans l'indication « réduction des événements thrombotiques chez des patients ayant un historique d'infarctus du myocarde ou de maladies artérielles périphériques ». L'utilisation de la 3-2-(chloro-phényl) -1-[4-(4-fluoro-benzyl)-pipérazin-1-yl]-propénone, un autre antagoniste de PAR-1, dans le domaine cardio-vasculaire, notamment comme antiagrégant plaquettaire, dans le traitement tant curatif que préventif de la thrombose artérielle ou veineuse, de l'angor stable, des troubles du rythme cardiaque, de l'infarctus du myocarde, de l'hypertension, de l'insuffisance cardiaque, des accidents vasculaires cérébraux, des syndromes coronaires aigus, a également été décrite dans l'art antérieur, illustré notamment par le document WO 2007/147824.

L'expression des récepteurs PAR, et notamment de PAR-1, dans le tractus digestif est décrite dans la littérature, notamment dans la publication de Vergnolle, 2004, dans J. Pharmacol, 141, 1264-1274.

Rien dans les travaux décrits dans cette publication, ni dans l'art antérieur en général, en particulier dans la publication précitée de Vergnolle et al., 2004, Journal of Clinical Investigation, 114(10): 1444-1456, ne laissait cependant supposer un rôle d'antagonistes spécifiques de ce récepteur vis-à-vis de la douleur et des dommages au niveau de l'épithélium intestinal impliqués dans le cadre de ces maladies, tel que les présents inventeurs l'ont maintenant découvert.

Ainsi, selon un premier aspect, la présente invention concerne un antagoniste de PAR-1, choisi dans le groupe constitué du vorapaxar, de l'atopaxar, de la 3-2-(chloro-phényl)-1-[4-(4-fluoro-benzyl)-pipérazin-1-yl]-propénone et de leurs sels pharmaceutiquement acceptables, pour son utilisation pour la prévention et/ou le traitement d'une maladie inflammatoire chronique de l'intestin et du côlon chez un sujet, et en particulier pour son utilisation pour soulager la douleur et/ou réparer les tissus épithéliaux de l'intestin chez ledit sujet atteint d'une maladie inflammatoire chronique de l'intestin et du côlon.

Ce sujet est notamment un mammifère, par exemple un mammifère non humain. Il s'agit préférentiellement d'un être humain.

On entend dans la présente description, par le terme traitement, un traitement curatif de la maladie, et plus particulièrement la diminution et/ou l'inhibition du développement d'au moins un des symptômes de la maladie, l'augmentation de la phase de rémission et/ou la diminution du nombre de crises ou de leur fréquence. L'antagoniste de PAR-1 selon la présente invention permet en particulier, entre autres, de soulager efficacement le symptôme de douleur lié à la maladie.

On entend, par le terme prévention, le fait de diminuer, ou même d'éviter totalement, l'apparition de la maladie.

On entend, par « réparer les tissus épithéliaux », de manière classique pour l'homme du métier, le fait de réduire l'hyperperméabilité épithéliale intestinale et de rétablir, au moins partiellement, la mécanique de l'intestin.

Par antagoniste du récepteur PAR-1 (également nommé antagoniste de PAR-1), on entend dans la présente description, de manière classique en elle-même, un composé qui interagit avec le récepteur PAR-1, pour l'espèce du sujet considéré, en s'opposant à l'effet de son ligand naturel, la thrombine, ou par des protéases. Généralement, les antagonistes de PAR-1 agissent par interaction directe avec le récepteur PAR-1 pour bloquer son activation.

La présente invention concerne notamment un antagoniste de PAR-1 pour son utilisation pour le traitement des maladies inflammatoires chroniques de l'intestin et du côlon chez un sujet en ayant besoin, c'est-à-dire atteint par une ou plusieurs de ces maladies, et plus particulièrement pour réduire la douleur et/ou réparer les tissus épithéliaux de l'intestin chez ce sujet, ledit antagoniste de PAR-1 étant administré audit sujet dans une quantité thérapeutiquement efficace.

La maladie inflammatoire chronique de l'intestin et du côlon visée par la présente invention peut aussi bien être la maladie de Crohn que la rectocolite hémorragique.

Le vorapaxar, l'atopaxar et la 3-2-(chloro-phényl)-1-[4-(4-fluoro-benzyl)-pipérazin-1-yl]-propénone sont des antagonistes connus de PAR-1, comme il a été indiqué ci-avant. Leur utilisation thérapeutique permet de prévenir et de traiter les MICls de manière particulièrement efficace, ce qui n'avait jamais été décrit ni suggéré dans l'art antérieur.

Le vorapaxar, également nommé ethyl N-[(1R,3aR,4aR,6R,8aR,9S,9aS)-9-[(E)-2-[5-(3-fluorophenyl)pyridin-2-yl]ethenyl]-1-methyl-3-oxo-3a,4,4a,5,6,7,8,8a,9,9a-decahydro-1H-benzo[f][2]benzofuran-6-yl]carbamate (SCH-530348, n° CAS 618385-01-6) répond à la formule (I) :

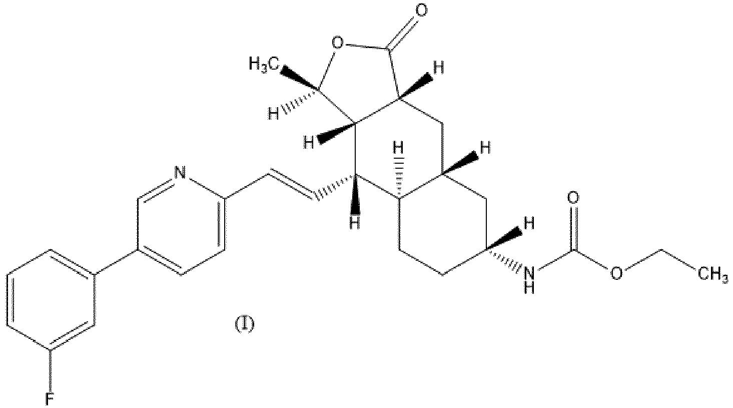

Ce composé est notamment décrit dans le document WO 03/089428. Il se présente généralement sous la forme d'un sel de sulfate.

L'atopaxar, ou 1-(3-tert-butyl-4-methoxy-5-morpholin-4-ylphenyl)-2-(5,6-diethoxy-4-fluoro-3-imino-1H-isoindol-2-yl)ethanone (E5555, SCH-602539, n° CAS 751475-53-3) répond à la formule (II) :

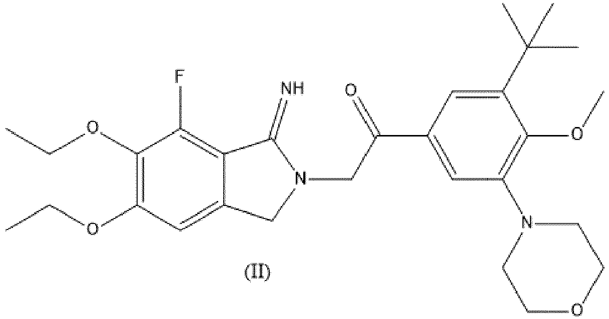

La 3-2-(chloro-phényl)-1-[4-(4-fluoro-benzyl)-pipérazin-1-yl]-propénone présente quant à elle la formule (III) :

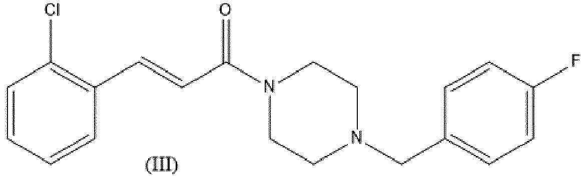

Par sel pharmaceutiquement acceptable, on entend dans la présente description tout sel desdits composés mettant en oeuvre, en tant que contre-ion, une espèce ne produisant aucun effet adverse, allergique ou autre réaction indésirable quand il est administré à un sujet, en particulier à un mammifère.

Tout sel conventionnel non toxique des antagonistes de PAR-1 selon l'invention, qu'il soit formé à partir d'acides organiques ou inorganiques, peut être mis en oeuvre selon l'invention. A titre d'exemple, on peut citer les sels dérivés d'acides inorganiques tels que les acides chlorhydrique, bromhydrique, phosphorique, sulfurique, et les sels dérivés d'acides organiques tels que les acides acétique, trifluoroacétique, propionique, succinique, fumarique, malique, tartarique, citrique, ascorbique, maléique, glutamique, benzoïque, salicylique, toluènesulfonique, méthanesulfonique, stéarique, lactique, etc.

Ces sels peuvent être synthétisés à partir d'un antagoniste de PAR-1 selon l'invention et des acides correspondants, selon toute méthode chimique classique en elle-même.

L'antagoniste de PAR-1 peut être utilisé tel quel, ou sous forme de solvate, par exemple dans l'eau ou l'éthanol.

Un autre aspect de l'invention concerne une composition pharmaceutique contenant un antagoniste de PAR-1 en tant que principe actif, et au moins un excipient pharmaceutiquement acceptable, l'antagoniste de PAR-1 étant choisi dans le groupe constitué du vorapaxar, de l'atopaxar, de la 3-2-(chloro-phényl)-1-[4-(4-fluoro-benzyl)-pipérazin-1-yl]-propénone et de leurs sels pharmaceutiquement acceptables, pour son utilisation, comme médicament, pour la prévention et/ou le traitement d'une maladie inflammatoire chronique de l'intestin et du côlon, et en particulier de la maladie de Crohn, chez un sujet, et plus particulièrement pour son utilisation pour soulager la douleur et/ou réparer les tissus épithéliaux de l'intestin chez ledit sujet atteint d'une maladie inflammatoire chronique de l'intestin et du côlon.

Ce sujet peut notamment être un mammifère, par exemple un mammifère non humain, ou de préférence un être humain.

La composition pharmaceutique selon l'invention peut contenir, outre l'antagoniste de PAR-1, tout excipient pharmaceutiquement acceptable.

On entend, par pharmaceutiquement acceptable, le fait que l'excipient ne présente aucun effet adverse, allergique ou autre réaction indésirable quand il est administré à un mammifère, notamment à un humain.

Un tel excipient peut être un diluant, un adjuvant ou toute autre substance classique en elle-même pour la constitution des médicaments, telle qu'un agent préservatif, de remplissage, désintégrant, mouillant, émulsifiant, dispersant, antibactérien ou antifongique, ou bien encore un agent permettant de retarder l'absorption et la résorption intestinale et digestive, etc., ou l'un quelconque de leurs mélanges.

La composition pharmaceutique selon l'invention peut en outre contenir un ou plusieurs autres agents actifs, agissant ou non en synergie avec l'antagoniste de PAR-1 selon l'invention, par exemple un agent antidouleur.

La composition pharmaceutique selon l'invention peut être formulée selon toute forme galénique, notamment une forme convenant à une administration chez les mammifères, et en particulier chez les êtres humains.

La composition pharmaceutique selon l'invention peut être administrée au sujet par voie orale, sublinguale, sous-cutanée, intramusculaire, intraveineuse, transdermique, locale ou rectale. Dans ce cas, le principe actif peut être administré sous formes unitaires, en mélange avec des supports pharmaceutiques classiques. Les formes unitaires appropriées comprennent les formes pour administration par voie orale telles que les comprimés, les gélules, les poudres, les granules et les solutions ou suspensions orales, les formes pour administration sublinguale et buccale, les formes pour administration sous-cutanée ou transdermique, topique, intramusculaire, intraveineuse, intra-nasale ou intraoculaire, et les formes pour administration intravésicale, intramurale ou rectale.

La composition pharmaceutique selon l'invention est de préférence administrée au sujet par voie orale. Elle présente alors une forme galénique adaptée à une telle administration par voie orale. Cette forme galénique peut être classique en elle-même.

La composition pharmaceutique selon l'invention peut être solide et se présenter par exemple sous forme de comprimés, de gélules ou de granules.

Des comprimés peuvent notamment être obtenus par mélange du principe actif avec un véhicule pharmaceutiquement compatible tel que la gélatine, l'amidon, le lactose, le stéarate de magnésium, le talc, la gomme arabique, la silice ou analogues. Les comprimés peuvent être enrobés de saccharose ou d'autres matières appropriées ou encore être traités de telle sorte qu'ils aient une activité prolongée ou retardée et qu'ils libèrent de façon continue, de préférence dans l'intestin et le côlon, une quantité prédéterminée de principe actif.

La composition pharmaceutique selon l'invention peut autrement se présenter sous forme de gélules, obtenues par mélange du principe actif avec un diluant et incorporation du mélange obtenu dans des gélules molles ou dures.

Des poudres et des granules, en particulier dispersibles dans l'eau, peuvent être formés par mélange du principe actif avec des agents de dispersion, des agents mouillants et/ou des agents de mise en suspension, de même qu'avec des correcteurs du goût ou des édulcorants.

La composition pharmaceutique selon l'invention peut alternativement se présenter sous forme fluide, notamment sous forme de sirop ou d'élixir contenant l'ingrédient actif conjointement avec un édulcorant, un antiseptique, ainsi qu'un agent donnant du goût et un colorant approprié.

La composition pharmaceutique selon l'invention peut autrement être administrée au sujet par voie rectale. Elle présente alors une forme galénique adaptée, par exemple celle d'un gel, d'une crème, d'une poudre, d'une suspension, d'une solution, d'une mousse ou d'un suppositoire, qui sont préparés avec des liants fondant à la température rectale, par exemple du beurre de cacao ou des polyéthylènes glycols.

La composition pharmaceutique selon l'invention peut autrement être administrée au sujet par voie parentérale (intraveineuse, intramusculaire, intradermique, sous-cutanée), intranasale, intraoculaire, intravésicale ou intramurale. Elle présente alors une forme galénique adaptée, par exemple celle d'une suspension aqueuse, d'une solution saline isotonique ou d'une solution stérile et injectable, qui contiennent des agents de dispersion et/ou des agents mouillants pharmaceutiquement compatibles.

Le principe actif peut autrement être formulé sous forme de microcapsules, éventuellement avec un ou plusieurs supports additifs.

Les formulations appropriées pour chaque forme d'administration particulière choisie sont connues par l'homme du métier et décrites, par exemple dans l'ouvrage de Remington, The Science and Practice of Pharmacy, 19ème édition, 1995, Mack Publishing Company.

La concentration de l'antagoniste de PAR-1 dans la composition pharmaceutique selon l'invention est de préférence choisie pour délivrer au sujet, à chaque administration, une quantité de cet antagoniste qui est efficace pour obtenir la réponse thérapeutique désirée, notamment le niveau de réduction de la douleur et/ou le niveau de réparation des tissus épithéliaux de l'intestin souhaités. Le niveau de dose thérapeutiquement efficace de l'antagoniste de PAR-1 spécifique pour un sujet particulier varie en fonction de nombreux facteurs tels que, par exemple, la pathologie exacte et sa gravité, la voie d'administration choisie, le poids corporel, l'âge, le sexe et la santé générale du sujet, la durée du traitement, les médicaments éventuellement utilisés en parallèle, la sensibilité de l'individu à traiter, etc.

En conséquence, la posologie optimale devra être déterminée par le spécialiste en la matière en fonction des paramètres qu'il juge pertinents.

Bien que les doses efficaces puissent varier dans de larges proportions, les doses journalières du principe actif selon l'invention pourraient s'échelonner entre 0,1 mg et 1000 mg par 24 heures, de préférence entre 1 et 100 mg par 24 heures et préférentiellement entre 1 et 10 mg par 24 heures, en une ou plusieurs prises, de préférence en une seule prise.

Préférentiellement, l'administration de la composition pharmaceutique selon l'invention est débutée au plus tôt dès le diagnostic de la maladie et, de préférence, dans les 12 premiers mois suivant l'événement aigu.

La présente invention s'exprime également sous forme d'un procédé de prévention et/ou de traitement d'une maladie inflammatoire chronique de l'intestin et du côlon, notamment de la maladie de Crohn, chez un sujet, et en particulier d'un procédé pour soulager la douleur et/ou réparer les tissus épithéliaux de l'intestin chez ledit sujet atteint d'une maladie inflammatoire chronique de l'intestin et du côlon. Le sujet peut être, en particulier, un mammifère et préférentiellement un être humain. Ce procédé comprend l'administration audit sujet d'une quantité thérapeutiquement efficace d'un antagoniste de PAR-1 tel que défini ci-avant, en particulier le vorapaxar ou un de ses sels pharmaceutiquement acceptables, ou d'une composition pharmaceutique contenant un antagoniste de PAR-1 tel que défini ci-avant et au moins un excipient pharmaceutiquement acceptable.

Ce procédé peut répondre à l'une ou plusieurs des caractéristiques décrites ci-avant en référence à l'utilisation de l'antagoniste de PAR-1 et/ou de la composition pharmaceutique le contenant.

Dans les modes de réalisation de l'invention dans lesquels la composition pharmaceutique selon l'invention, contenant un antagoniste de PAR-1 tel que défini ci-avant en tant que principe actif et au moins un excipient pharmaceutiquement acceptable, présente une forme galénique solide adaptée à une administration par voie orale, l'antagoniste de PAR-1 peut être inclus dans un coeur recouvert d'un enrobage gastro-résistant.

On entend dans la présente description, par gastro-résistant, de manière classique en elle-même, le fait que cet enrobage interdit toute libération du principe actif avant d'avoir atteint l'intestin et le côlon, en empêchant ainsi notamment tout contact direct entre le principe actif et la muqueuse gastrique. Ainsi, l'enrobage gastro-résistant ne se dégrade pas dans la partie supérieure du tractus intestinal, qui a une acidité élevée, et il permet une libération du principe actif essentiellement dans l'iléon distal et le côlon, où le pH intestinal augmente progressivement à des valeurs allant de 5 à 7.

La composition pharmaceutique selon l'invention peut par exemple se présenter sous forme de gélule contenant une dispersion homogène du principe actif dans une matrice hydrophile, par exemple comprenant un dérivé de la cellulose, l'ensemble formant un coeur qui est recouvert d'un enrobage par exemple à base d'éthylcellulose ou à base d'un polymère dérivé d'esters d'acide acrylique et d'acide méthacrylique, tel que les polymères commercialisés sous la dénomination commerciale Eudragit^{®}.

Une telle gélule peut par exemple contenir entre 1 et 10 mg, par exemple environ 2,5 mg, de l'antagoniste de PAR-1. Elle peut par exemple être administrée par voie orale, à raison d'une gélule par jour.

Des exemples de formes galéniques conformes à l'invention, pour administration par voie orale et visant une libération entérique du principe actif, sont notamment décrits dans les documents WO 2009/114773, US 2015/0196518 ou encore WO 2005/193788, l'enseignement de ces documents étant transposable à l'antagoniste de PAR-1 selon l'invention.

La présente invention s'exprime également dans les termes de l'utilisation d'un antagoniste de PAR-1 choisi dans le groupe constitué du vorapaxar, de l'atopaxar, de la 3-2-(chloro-phényl)-1-[4-(4-fluoro-benzyl)-pipérazin-1-yl]-propénone et de leurs sels pharmaceutiquement acceptables, pour la fabrication d'un médicament pour le traitement et/ou la prévention d'une maladie inflammatoire chronique de l'intestin et du côlon, et en particulier pour réduire la douleur et/ou réparer les tissus épithéliaux de l'intestin chez un sujet atteint d'une maladie inflammatoire chronique de l'intestin et du côlon, en particulier de la maladie de Crohn.

Cette utilisation peut répondre à l'une ou plusieurs des caractéristiques décrites ci-avant en référence à l'antagoniste de PAR-1 et à la composition pharmaceutique selon l'invention.

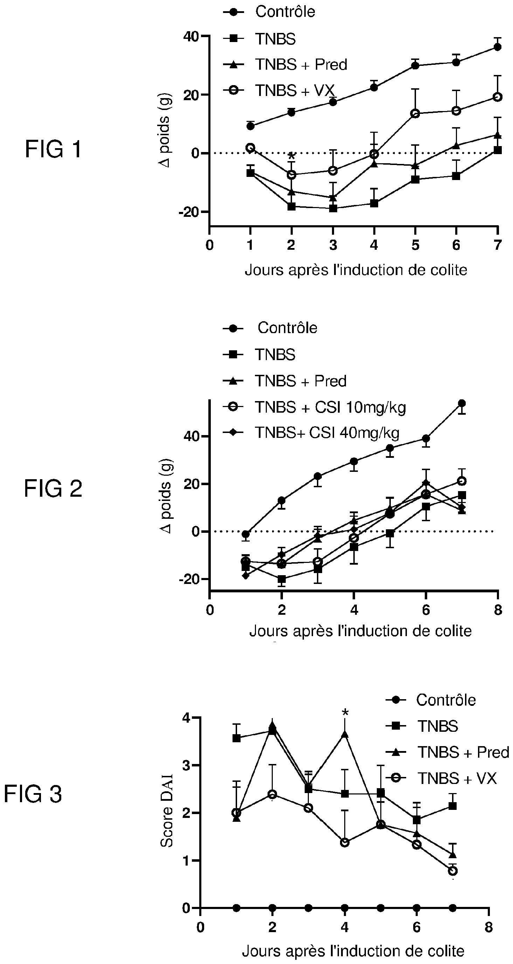

Les caractéristiques et avantages de l'invention apparaîtront plus clairement à la lumière des exemples de mise en oeuvre ci-après, fournis à simple titre illustratif et nullement limitatifs de l'invention, avec l'appui des figures 1 à 12, dans lesquelles :
- la figure 1 montre un graphe illustrant l'évolution du poids corporel (Δ poids) de rats chez lesquels une colite a été induite par le TNBS, en fonction du temps après l'induction de la colite, pour des rats non traités (« TNBS »), pour des rats traités quotidiennement avec la prednisolone (« TNBS + Pred ») et pour des rats traités quotidiennement avec le vorapaxar (« TNBS + VX »), le contrôle représentant des rats non traités et chez lesquels aucune colite n'a été induite ;
- la figure 2 montre un graphe illustrant l'évolution du poids corporel (Δ poids) de rats chez lesquels un colite a été induite par le TNBS, en fonction du temps après l'induction de la colite, pour des rats non traités (« TNBS »), pour des rats traités quotidiennement avec la prednisolone (« TNBS + Pred ») et pour des rats quotidiennement traités avec le composé selon l'invention CSI (« TNBS + CSI »), le CSI étant administré à des doses de 10 ou de 40 mg/kg/jour, le contrôle représentant des rats non traités et chez lesquels aucune colite n'a été induite ;
- la figure 3 montre un graphe illustrant l'évolution du score DAI de rats chez lesquels une colite a été induite par le TNBS, en fonction du temps après l'induction de la colite, pour des rats non traités (« TNBS »), pour des rats traités quotidiennement avec la prednisolone (« TNBS + Pred ») et pour des rats traités quotidiennement avec le vorapaxar (« TNBS + VX »), le contrôle représentant des rats non traités et chez lesquels aucune colite n'a été induite ;

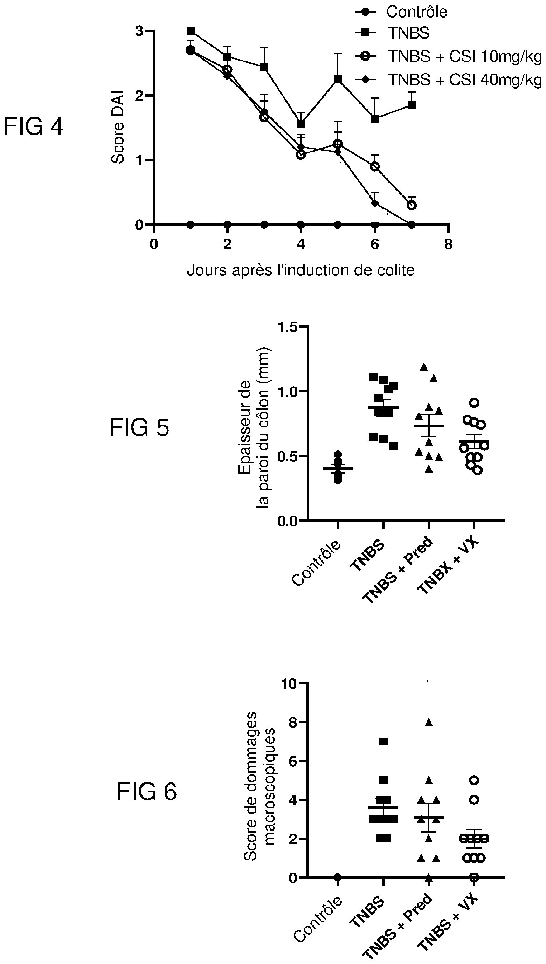

- la figure 4 montre un graphe illustrant l'évolution du score DAI de rats chez lesquels une colite a été induite par le TNBS, en fonction du temps après l'induction de la colite, pour des rats non traités (« TNBS ») et pour des rats traités quotidiennement avec le composé selon l'invention CSI (« TNBS + CSI »), le CSI étant administré à des doses de 10 ou 40 mg/kg/jour, le contrôle représentant des rats non traités et chez lesquels aucune colite n'a été induite ;
- la figure 5 montre un graphe illustrant l'épaisseur de la paroi du côlon de rats chez lesquels une colite a été induite par le TNBS, 7 jours après l'induction de colite, pour des rats non traités (« TNBS »), pour des rats traités quotidiennement avec la prednisolone (« TNBS + Pred ») et pour des rats traités quotidiennement avec le vorapaxar (« TNBS + VX »), le contrôle représentant des rats non traités et chez lesquels aucune colite n'a été induite ;
- la figure 6 montre un graphe illustrant le score de dommages macroscopiques chez des rats chez lesquels une colite a été induite par le TNBS, 7 jours après l'induction de colite, pour des rats non traités (« TNBS »), pour des rats traités quotidiennement avec la prednisolone (« TNBS + Pred ») et pour des rats traités quotidiennement avec le vorapaxar (« TNBS + VX »), le contrôle représentant des rats non traités et chez lesquels aucune colite n'a été induite ;

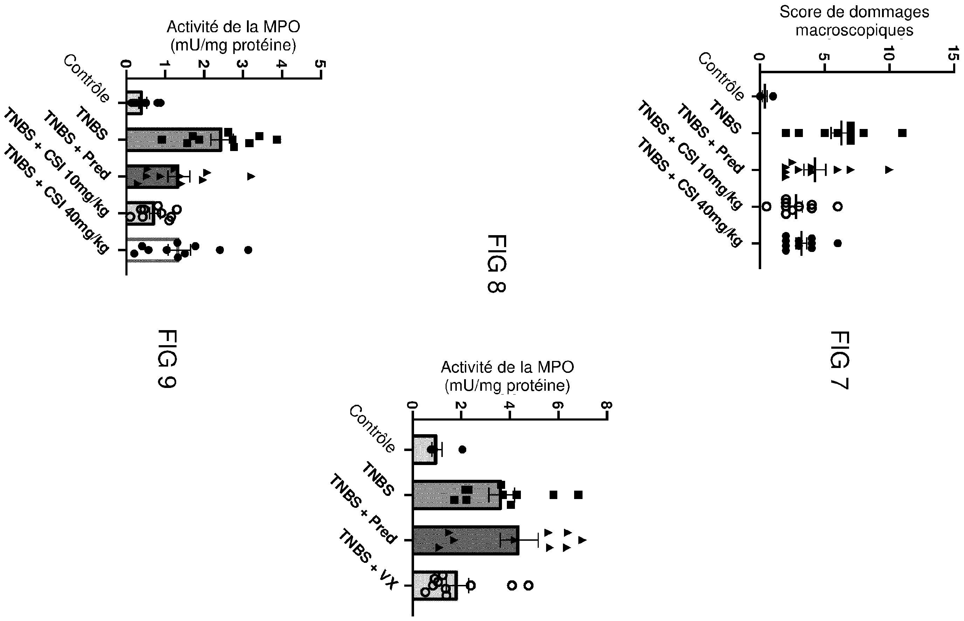

- la figure 7 montre un graphe illustrant le score de dommages macroscopiques chez des rats chez lesquels une colite a été induite par le TNBS, 7 jours après l'induction de colite, pour des rats non traités (« TNBS »), pour des rats traités quotidiennement avec la prednisolone (« TNBS + Pred ») et pour des rats traités quotidiennement avec le composé selon l'invention CSI (« TNBS + CSI »), le CSI étant administré à des doses de 10 ou 40 mg/kg/jour, le contrôle représentant des rats non traités et chez lesquels aucune colite n'a été induite ;
- la figure 8 montre un graphe illustrant l'activité de la myéloperoxydase (MPO) chez des rats chez lesquels une colite a été induite par le TNBS, 7 jours après l'induction de colite, pour des rats non traités (« TNBS »), pour des rats traités quotidiennement avec la prednisolone (« TNBS + Pred ») et pour des rats traités quotidiennement avec le vorapaxar (« TNBS + VX »), le contrôle représentant des rats non traités et chez lesquels aucune colite n'a été induite ;
- la figure 9 montre un graphe illustrant l'activité de la myéloperoxydase (MPO) chez des rats chez lesquels une colite a été induite par le TNBS, 7 jours après l'induction de colite, pour des rats non traités (« TNBS »), pour des rats traités quotidiennement avec la prednisolone (« TNBS + Pred ») et pour des rats traités quotidiennement avec le composé selon l'invention CSI (« TNBS + CSI »), le CSI étant administré à des doses de 10 ou 40 mg/kg/jour, le contrôle représentant des rats non traités et chez lesquels aucune colite n'a été induite ;

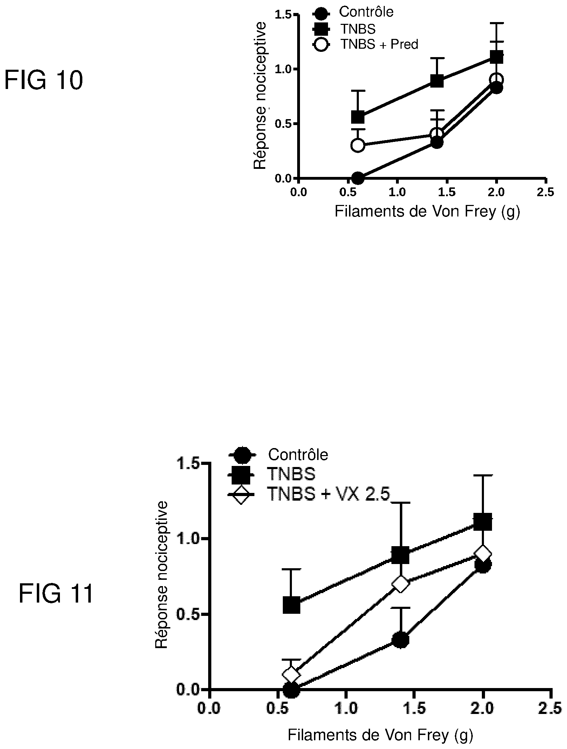

- la figure 10 montre un graphe illustrant l'évolution de la réponse nociceptive induite par le TNBS chez le rat, en fonction de la masse de filaments de Von Frey, 7 jours après l'induction de colite, pour des rats non traités (« TNBS ») et pour des rats traités quotidiennement avec la prednisolone (« TNBS + Pred »), le contrôle représentant des rats non traités et chez lesquels aucune colite n'a été induite ;
- la figure 11 montre un graphe illustrant l'évolution de la réponse nociceptive induite par le TNBS chez le rat, en fonction de la masse de filaments de Von Frey, 7 jours après l'induction de colite, pour des rats non traités (« TNBS ») et pour des rats traités quotidiennement avec le vorapaxar (« TNBS + VX »), le contrôle représentant des rats non traités et chez lesquels aucune colite n'a été induite ;

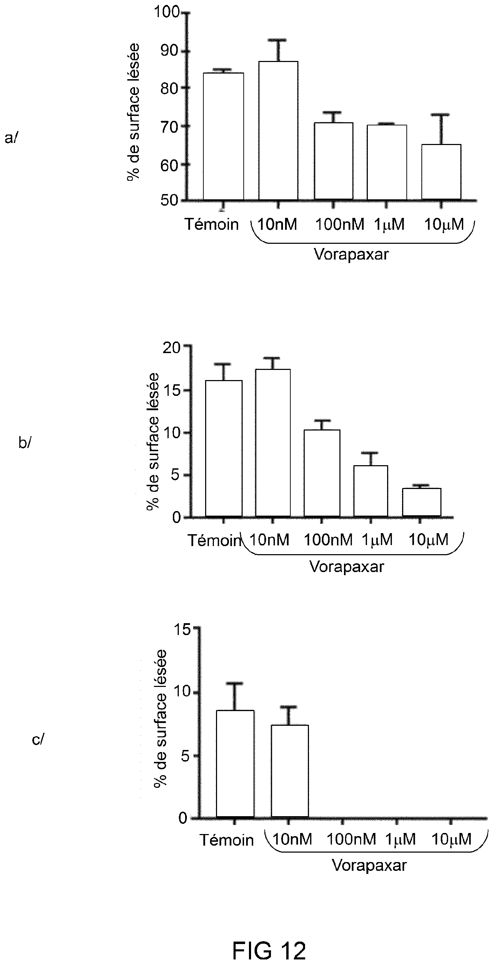

- et la figure 12 montre des graphes représentant le pourcentage de surface lésée pour des cellules épithéliales de côlon humain sur lesquelles a été pratiquée une lésion, mises en contact avec du vorapaxar à différentes concentrations ou avec le véhicule seul (« Témoin »), respectivement a/ 4 h après la lésion, b/ 22 h après la lésion et c/ 28 h après la lésion.

### Exemple 1 : évaluation d'effets thérapeutiques potentiels d'antagonistes de PAR-1 dans un modèle de maladie intestinale inflammatoire chez le rat

Le but de cette étude est d'évaluer l'efficacité de deux antagonistes de PAR-1, le vorapaxar et la 3-2-(chloro-phényl)-1-[4-(4-fluoro-benzyl)-pipérazin-1-yl]-propénone (ci après désignée CSI) dans un modèle de maladie intestinale inflammatoire induite par le 2,4,6 acide trinitrobenzène sulfate (TNBS) chez le rat de laboratoire mâle de la lignée wistar. Ce modèle est décrit dans la publication de Whittle et al., 2003, dans Methods Mol. Biol., 225, 209-222.

Dans ce modèle de colite induite, la réaction inflammatoire est mesurée tous les jours et est bien installée 7 jours après administration intra colique de TNBS.

Différents paramètres sont mesurés : le poids des rats, la présence de sang dans les fèces et les diarrhées évalués par l'indice d'activité de la maladie (score DAI, pour l'anglais « disease activity index), l'intensité de la douleur (évaluée par la technique des filaments de Von Frey mesurée les jours 3 et 7).

Les rats sont sacrifiés 7 jours après le début du traitement et les dommages macroscopiques, l'oedème et un marqueur de l'inflammation, la myéloperoxydase (MPO), sont quantifiés.

### Protocole

Les essais avec les antagonistes de PAR-1 sont réalisés en deux études, portant respectivement sur le vorapaxar et sur le CSI.

La rectocolite est induite par une administration intra colique de 2,4,6 acide trinitrobenzène sulfate (TNBS) (30 mg de TNBS dans 0,25 ml d'une solution 50% éthanol/NaCl) *via* un cathéter (polyéthylène (PE)-60) inséré à 8 cm de proximal de l'anus.

4 groupes de 10 rats reçoivent une administration intra colique de TNBS, ainsi qu'un traitement *per os* de vorapaxar à la dose de 2,5 mg/kg/jour ou de CSI aux doses de 10 et 40 mg/kg/jour ou de prednisolone (composé proposé par l'art antérieur pour le traitement des MICIs) à la dose de 3 mg/kg/jour. Les traitements *per os* débutent 1 h avant l'administration intra colique de TNBS.

A titre d'exemple comparatif négatif, 1 groupe de 10 rats reçoit une administration intra colique de TNBS et de véhicule du vorapaxar ( tampon phosphate salin - PBS) ou de véhicule du CSI (carboxyméthyl-cellulose - CMC à 1 %) en *per os* tous les jours.

1 groupe de 6 rats contrôle reçoit par ailleurs une administration intra colique de NaCl en lieu et place du TNBS.

Pour la 1^{ère} étude, portant sur le vorapaxar, 4 groupes de rats sont traités comme indiqué dans le tableau 1 ci-dessous.

| Groupe | Administration intra colique | Traitement per os | Dose du traitement per os / 24h | Nombre d'individus du groupe |
|---|---|---|---|---|
| 1 | NaCl | - | - | 6 |
| 2 | TNBS | PBS | - | 10 |
| 3 | TNBS | vorapaxar | 2,5 mg/kg | 10 |
| 4 | TNBS | prednisolone | 3 mg/kg | 10 |

Pour la 2^{ème} étude, portant sur le composé CSI, 5 groupes de rats sont traités comme indiqué dans le tableau 2 ci-dessous.

| Groupe | Administration intra colique | Traitement per os | Dose du traitement per os / 24h | Nombre d'individus du groupe |
|---|---|---|---|---|
| 1 | NaCl | - | - | 6 |
| 2 | TNBS | CMC 1% | - | 10 |
| 3 | TNBS | CSI | 10 mg/kg | 10 |
| 4 | TNBS | CSI | 40 mg/kg | 10 |
| 5 | TNBS | prednisolone | 3 mg/kg | 10 |

Le score DAI est déterminé comme suit. Le développement de la maladie est estimé tous les jours. La consistance des selles, ainsi que la présence de sang dans les fèces évaluée avec le test au papier Hemoccult^{®}, sont respectivement quantifiées par les échelles de sévérité du tableau 3 ci-dessous. Les valeurs obtenues sont additionnées pour obtenir le score DAI.

| Echelle de consistance des selles | | Score Hemoccult^{®} | |
|---|---|---|---|
| 0 | Normal | 0 | Normal |
| 0,5 | Selles légèrement molles | 0,5 | Pas de sang visible et Hemoccult^{®} ± |
| 1 | Selles molles | 1 | Pas de sang visible et Hemoccult^{®} + |
| 1,5 | Diarrhée faible | 1,5 | Pas de sang visible et Hemoccult^{®} ++ |
| 2 | Diarrhée | 2 | Sang visible |

Le score de dommages macroscopiques est évalué selon le protocole décrit dans la publication de Cornera et al., 1999, Dig. Dis. Sci. 44: 1448-1457, comme suit. Au jour 7 après induction de la colite, les rats sont sacrifiés par inhalation de dioxyde de carbone CO₂, le côlon (de l'anus au Caecum) est prélevé et les dommages macroscopiques sont évalués selon les paramètres observés et présentés dans le tableau 4 ci-dessous, pour déterminer le score des dommages macroscopiques.

| Caractéristique | Score |
|---|---|
| Apparence normale | 0 |
| Hyperhémie, pas d'ulcère | 1 |
| Ulcération sans hyperhémie ou épaississement de la paroi intestinale | 2 |
| Ulcération avec inflammation sur 1 zone | 3 |
| Ulcération/inflammation sur 2 zones ou plus | 4 |
| Zones de dommages majeurs étendues de >1 cm sur la longueur du côlon | 5 |
| Zones de dommages majeurs étendues de >2 cm sur la longueur du côlon - le score est majoré de 1 pour chaque cm additionnel endommagé | 6-10 |

### Résultats

Des signes cliniques significatifs et représentatifs apparaissent dans ce modèle de colite induite par le TNBS, tels que des diarrhées et la présence de sang dans les fèces (DAI) dès le début de l'étude. Ensuite une chronicité s'installe quotidiennement, évaluée par les différents paramètres ci-après.

L'évolution du poids corporel des rats en fonction du temps est montrée sur la figure 1 pour le vorapaxar et sur la figure 2 pour le CSI. Ces résultats démontrent qu'une perte de poids significative apparait dès le jour 1 après l'injection de TNBS (« TNBS ») par rapport aux rats naïfs, n'ayant pas subi d'induction de colite (« Contrôle »), qui ont un gain de poids quotidien. Le vorapaxar, réduit significativement cette perte de poids dès le 2^{ème} jour. Le CSI, comme la prednisolone, atténuent cette perte de poids.

L'évolution du score DAI en fonction du temps est montrée sur la figure 3 pour le vorapaxar et sur la figure 4 pour le CSI.

L'induction de la colite provoque chez les rats différents signes cliniques soutenus tels que des diarrhées et la présence de sang dans les fèces (score DAI) chez les animaux ayant été soumis au TNBS (« TNBS ») par rapport au groupe de rats naïfs (« Contrôle »). Ceci montre clairement que l'administration de TNBS reproduit tous les paramètres de la colite.

L'administration orale quotidienne de prednisolone à la dose de 3 mg/kg ne prévient pas l'augmentation de l'indice d'activité de la maladie (DAI) à tous les points observés. Ce résultat démontre que les rats soumis au TNBS sont fortement atteints et qu'il est difficile d'améliorer leurs symptômes.

En revanche, l'administration quotidienne de 2,5 mg/kg du vorapaxar ou de 10 mg/kg du composé CSI réduit significativement l'indice d'activité de la maladie au 7^{ème} jour. L'indice d'activité de la maladie est réduit significativement dès le 1^{er} jour du traitement avec le vorapaxar à 2,5 mg/kg en comparaison avec le groupe véhicule (« TNBS »). Ces résultats démontrent clairement que les antagonistes de PAR-1 selon l'invention réduisent les paramètres de la colite dans ce modèle.

L'induction de la rectocolite entraine par ailleurs l'apparition d'oedème, qui est quantifié par la mesure de l'épaisseur macroscopique du tissu de la paroi du côlon.

L'effet sur l'épaisseur des tissus du vorapaxar est montré sur la figure 5.

Comme on peut l'observer, l'administration orale quotidienne de prednisolone à la dose de 3 mg/kg ne prévient pas l'apparition de l'oedème. Ce résultat démontre là aussi que les rats auxquels a été administré le TNBS sont fortement atteints et qu'il est difficile d'améliorer leurs symptômes.

En revanche, l'administration quotidienne à 2,5 mg/kg de vorapaxar réduit significativement l'apparition de cet oedème au 7^{ème} jour. Ainsi, l'épaisseur de la paroi du côlon représentative de l'apparition de l'oedème dans le tissu intestinal est significativement réduite avec le traitement par le vorapaxar à 2,5 mg/kg/jour, en comparaison au groupe véhicule (« TNBS »).

Les effets des antagonistes de PAR-1 sur les paramètres inflammatoires sont évalués par détermination du score de dommages macroscopiques et par quantification de l'activité du marqueur myéloperoxydase (MPO).

L'effet sur le score de dommages macroscopiques est montré sur la figure 6 pour le vorapaxar et sur la figure 7 pour le CSI.

L'effet sur l'activité de la MPO est montré sur la figure 8 pour le vorapaxar et sur la figure 9 pour le CSI.

Sept jours après le début de l'administration de TNBS (« TNBS »), le score de dommages macroscopiques est significativement plus élevé comparé à celui obtenu avec les rats naïfs (« Contrôle »). Un examen macroscopique du côlon des rats soumis au TNBS montre qu'il présente des érythèmes marqués et la présence d'oedèmes et d'ulcères.

La myéloperoxydase (MPO), exprimée majoritairement dans des granulocytes, est un marqueur de l'inflammation. La présence de lésions des muqueuses, associées à une infiltration leucocytaire, résulte en une élévation de l'activité de la MPO.

En comparaison du groupe véhicule (« TNBS »), le traitement par la prednisolone à 3 mg/kg réduit significativement le score de dommages macroscopiques ; de même l'examen macroscopique des côlons de ces rats permet de conclure que les érythèmes, les oedèmes et les ulcères sont moins marqués que ceux observés dans le groupe véhicule (« TNBS »).

En comparaison du groupe véhicule (« TNBS »), le traitement par le vorapaxar et par le CSI aux doses testées réduit significativement le score de dommages macroscopiques. L'examen macroscopique des côlons montre également une forte réduction des érythèmes, oedèmes et ulcères.

De la même façon, les traitements avec la prednisolone et avec les antagonistes de PAR-1 selon l'invention, aux doses testées, réduisent significativement l'activité de la MPO par rapport au groupe véhicule (« TNBS »). A la dose de 40 mg/kg/jour du composé CSI, on observe que cette activité est fortement diminuée.

Ces résultats démontrent clairement que la colite induite par le TNBS provoque une augmentation significative des paramètres inflammatoires (dommages macroscopiques et activité de la MPO), cette augmentation étant significativement réduite par les antagonistes de PAR-1 testés.

L'intensité de la douleur est mesurée par la technique des filaments de Von Frey aux jours 1, 3 et 7, selon le protocole décrit dans la publication de Auge et al., 2013, Eur. J. Pharmacol. 707: 32-40.

L'effet sur la réponse nociceptive, mesurée par la technique des filaments de Von Frey en fonction de la masse de filaments de Von Frey, est montré sur la figure 10 pour la prednisolone et sur la figure 11 pour le vorapaxar.

On y observe que le vorapaxar à la dose de 2,5 mg/kg/jour inhibe significativement la réponse de type allodynie. En comparaison, la prednisolone induit uniquement une inhibition partielle.

Ces résultats démontrent que la colite induite par le TNBS provoque une augmentation significative de la réponse nociceptive (allodynie), cette augmentation étant significativement réduite par le vorapaxar.

### Exemple 2 : évaluation d'effets thérapeutiques potentiels d'un antagoniste de PAR-1 dans un modèle de maladie intestinale inflammatoire chez la souris

Le but de cette étude est d'évaluer l'efficacité d'un antagoniste de PAR-1 selon l'invention, la 3-2-(chloro-phényl)-1-[4-(4-fluoro-benzyl)-pipérazin-1-yl]-propénone (CSI) dans un modèle de maladie intestinale inflammatoire, le Dextran Sulfate de sodium (DSS) chez la souris. Ce modèle DSS est décrit dans la publication de Choi et al., 2010, dans J. Biomed. Biotechnol., 2010:943516 ; doi: 10.1155/2010/943516.

Ce modèle présente un profil de cytokines Th1 dans sa phase aiguë, des périodes de rémission et des rechutes, il est par conséquent très similaire à la rectocolite ulcéreuse. Le DSS distribué dans l'eau de boisson peut provoquer une inflammation du tube digestif et produire des tumeurs colorectales chez le rongeur. Le DSS est très utilisé en tant que modèle animal des atteintes inflammatoires humaines du tube digestif.

La présente étude est menée chez des souris (C57BI6) âgées de 7 semaines. L'acide 5-aminosalicylique sert d'exemple comparatif positif.

Le premier jour de l'étude commence avec l'administration aux souris de boisson contenant le DSS à 5 % p/p dans l'eau, quotidienne jusqu'au jour du sacrifice. Le développement de la maladie est estimé tous les jours, avec des pesées précises des souris, l'évaluation de la consistance des selles ainsi que de la présence de sang dans les fèces par le test au papier Hemoccult^{®}. Le score DAI est calculé comme indiqué dans l'Exemple 1.

Des groupes de souris reçoivent chaque jour en per os le CSI à différentes concentrations, ou de l'acide 5-aminosalicylique (5-ASA), ou encore le véhicule seul, c'est-à-dire la carboxyméthyl-cellulose (CMC) à 1 %.

Les souris sont sacrifiées 7 jours après le début du traitement. Le côlon est excisé et les dommages macroscopiques sont évalués. Le côlon est alors coupé en 4 morceaux, la partie distale est placée dans un tube Eppendorf et stockée à -80 °C jusqu'à la mesure de l'activité de la myéloperoxydase et la quantification de protéines. La partie proximale est conservée dans la formaline à 10% pour de futures analyses histologiques. Les deux autres parties centrales sont placées dans des tubes Eppendorf et stockées à -80 °C pour des analyses futures.

### Protocole

Les souris sont réparties dans 5 groupes comme indiqué dans le tableau 5 ci-dessous.

| Groupe | Administration de DSS | Traitement | Dose du traitement / 24h | Nombre d'individus du groupe |
|---|---|---|---|---|
| 1 | 0% | - | - | 8 |
| 2 | 5% | CSI | 10 mg/kg | 10 |
| 3 | 5% | CSI | 40 mg/kg | 10 |
| 4 | 5% | CMC seul | 100 µl | 10 |
| 5 | 5% | 5-ASA | 50 mg/kg | 10 |

### Résultats

La variation du poids corporel des souris par rapport au jour 0 est montrée pour chaque groupe dans le tableau 6 ci-dessous. Dans ce tableau, « Moy » signifie la moyenne obtenue pour les différents individus du groupe, et « esm » représente l'écart standard à la moyenne.

| | Variation du poids corporel des souris par rapport au jour J0 (g) | | | | | | | |
|---|---|---|---|---|---|---|---|---|
| Groupe | Moy/esm | J1-J0 | J2-J0 | J3-J0 | J4-J0 | J5-J0 | J6-J0 | J7-J0 |
| 1 | Moy | 0,54 | 0,70 | 1,14 | 1,21 | 1,16 | 1,19 | 1,17 |
| | esm | 0,43 | 0,70 | 0,32 | 0,32 | 0,65 | 0,49 | 0,54 |
| 2 | Moy | 0,67 | 0,44 | 0,45 | -0,42 | -2,15 | -3,79 | -5,19 |
| | esm | 0,42 | 0,38 | 0,34 | 0,36 | 0,79 | 1,05 | 1,59 |
| 3 | Moy | -0,08 | -0,07 | -0,19 | -0,68 | -1,07 | -2,69 | -4,00 |
| | esm | 1,25 | 1,70 | 2,00 | 2,05 | 0,61 | 0,60 | 0,92 |
| 4 | Moy | 0,32 | 0,29 | 0,38 | -0,35 | -1,89 | -3,82 | -4,80 |
| | esm | 0,45 | 0,80 | 0,51 | 0,57 | 0,67 | 0,83 | 1,06 |
| 5 | Moy | 0,64 | 0,64 | 1,05 | -0,03 | -1,67 | -3,68 | -4,76 |
| | esm | 0,53 | 0,42 | 0,40 | 0,64 | 0,54 | 0,85 | 1,33 |

L'évolution de l'indice d'activité de la maladie des souris au cours du temps est montrée pour chaque groupe dans le tableau 7 ci-dessous. Dans ce tableau, « Moy » signifie la moyenne obtenue pour les différents individus du groupe, et « esm » représente l'écart standard à la moyenne.

| | Indice d'activité de la maladie | | | | | | | |
|---|---|---|---|---|---|---|---|---|
| Groupe | Moy/esm | J1 | J2 | J3 | J4 | J5 | J6 | J7 |
| 1 | Moy | 0,00 | 0,00 | 0,00 | 0,00 | 0,00 | 0,00 | 0,00 |
| | esm | 0,00 | 0,00 | 0,00 | 0,00 | 0,00 | 0,00 | 0,00 |
| 2 | Moy | 1,40 | 1,35 | 2,00 | 3,75 | 4,00 | 4,00 | 2,90 |
| | esm | 0,55 | 0,47 | 0,76 | 0,50 | 0,00 | 0,00 | 0,57 |
| 3 | Moy | 2,00 | 1,10 | 1,50 | 2,80 | 4,00 | 4,00 | 3,00 |
| | esm | 1,00 | 0,55 | 0,50 | 1,10 | 0,00 | 0,00 | 0,71 |
| 4 | Moy | 1,57 | 1,50 | 2,60 | 3,83 | 4,00 | 4,00 | 3,90 |
| | esm | 0,45 | 0,50 | 0,55 | 0,41 | 0,00 | 0,00 | 0,32 |
| 5 | Moy | 1,64 | 1,25 | 1,92 | 3,50 | 4,00 | 4,00 | 3,20 |
| | esm | 0,75 | 0,52 | 0,20 | 0,58 | 0,00 | 0,00 | 0,79 |

Les souris soumises au DSS (Groupe 4) développent des rectocolites, ce qui est démontré par une perte de poids et l'index d'activité de la maladie qui est fortement augmenté.

La perte de poids des souris soumises au DSS est présente dès le 4^{ème} jour et est significative pour les jours 5, 6 et 7 par rapport aux souris non soumises au DSS (Groupe 1).

L'indice d'activité de la maladie est très rapidement différent chez les souris soumises au DSS (Groupe 4) par rapport aux souris contrôles (Groupe 1), comme il est montré dans le tableau 7.

L'ensemble de ces résultats démontre clairement que l'administration de DSS reproduit tous les paramètres de la rectocolite.

L'administration orale quotidienne de 5-ASA à la dose de 50 mg/kg (Groupe 5) ne prévient pas la perte de poids ni l'augmentation de l'indice d'activité de la maladie à tous les points observés. Ce résultat démontre que les souris soumises au DSS sont fortement atteintes et qu'il est difficile d'améliorer leurs symptômes.

En revanche, l'administration quotidienne à 10 mg/kg de CSI (Groupe 2), réduit significativement l'indice d'activité de la maladie au 7^{ème} jour. A la dose de 40 mg/kg (Groupe 3), l'indice d'activité de la maladie est réduit significativement au 4^{ème} et au 7^{ème} jour du traitement en comparaison au groupe véhicule (Groupe 4). De plus, à cette dose le composé CSI selon l'invention réduit significativement la perte de poids au 6^{ème} jour. Ces résultats démontrent un effet protecteur de ce composé dans ce modèle de rectocolite chez la souris.

Au jour 7, après sacrifice des animaux, le côlon est prélevé. Le score de dommages macroscopiques, évalué comme décrit dans l'Exemple 1, et l'activité de la myéloperoxydase (MPO) sont déterminés. Les résultats obtenus sont résumés dans le tableau 8 ci-dessous.

| Groupe | Moy/esm | Score de dommages macroscopiques | Activité MPO (mU/mg protéines) |
|---|---|---|---|
| 1 | Moy | 0,0 | 0,80 |
| | esm | 0,0 | 0,45 |
| 2 | Moy | 5,2 | 12,99 |
| | esm | 0,7 | 3,93 |
| 3 | Moy | 4,0 | 6,9 |
| | esm | 0,6 | 2,5 |
| 4 | Moy | 9,6 | 10,97 |
| | esm | 0,5 | 1,68 |
| 5 | Moy | 6,5 | 9,34 |
| | esm | 0,8 | 3,17 |

Sept jours après le début du traitement avec le DSS (Groupe 4), le score de dommages macroscopiques est significativement plus élevé comparé à celui obtenu avec les souris contrôles (Groupe 1). Un examen macroscopique du côlon des souris soumises au DSS présente des érythèmes marqués et la présence d'oedèmes et d'ulcères. L'infiltration de cellules inflammatoires est estimée par la mesure de l'activité de la MPO, un index d'infiltration granulocytaire. L'administration de DSS résulte en une augmentation très importante et statistiquement significative de l'activité de la MPO par rapport aux souris témoins.

En comparaison du groupe véhicule (Groupe 4), le traitement par la 5-ASA à 50 mg/kg (Groupe 5) réduit significativement le score de dommages macroscopiques ; de même l'examen macroscopique des côlons de ces souris permet de conclure que les érythèmes, les oedèmes et les ulcères sont moins marqués que ceux observés dans le groupe véhicule.

En comparaison du groupe véhicule (Groupe 4), le traitement par le composé CSI selon l'invention aux deux doses testées (Groupes 2 et 3) réduit significativement le score de dommages macroscopiques. L'examen macroscopique des côlons montre également une forte réduction des érythèmes, oedèmes et ulcères.

A la dose de 40 mg/kg du composé CSI selon l'invention (Groupe 3), l'activité de la MPO est en outre fortement diminuée.

Ces résultats montrent que la 3-2-(chloro-phényl)-1-[4-(4-fluoro-benzyl)-pipérazin-1-yl]-propénone réduit de façon significative et dose-dépendante les signes quotidiens de rectocolite, et apparait plus efficace que la 5-ASA.

Les résultats présentés ci-avant dans l'Exemple 1 et l'Exemple 2 démontent bien que le traitement avec un antagoniste de PAR-1, qu'il s'agisse du vorapaxar ou de la 3-2-(chloro-phényl)-1-[4-(4-fluoro-benzyl)-pipérazin-1-yl]-propénone, réduit significativement l'inflammation et la douleur dans deux modèles de maladie de Crohn induite, respectivement chez le rat et la souris.

### Exemple 3 : formulations galéniques

Des exemples de compositions pharmaceutiques selon l'invention, se présentant sous une forme galénique adaptée à une administration par voie orale, pour une libération du principe actif essentiellement dans l'iléon distal et le côlon, sont décrits ci-après.

### Formule 1

La composition pharmaceutique selon l'invention se présente sous forme de microgranules contenant du vorapaxar, de l'atopaxar ou de la 3-2-(chloro-phényl)-1-[4-(4-fluoro-benzyl)-pipérazin-1-yl]-propénone, en mélange avec les excipients suivants : cellulose microcristalline, stéarate de magnésium.

Ces microgranules sont enrobés d'une pellicule semi-perméable d'éthylcellulose qui permet la diffusion des molécules actives présentes dans les microgranules.

Ils sont présentés sous forme de comprimés contenant chacun une quantité comprise entre 1 et 10 mg de principe actif et entre 0,02 et 1,2 mg d'éthylcellulose.

### Formule 2

La composition pharmaceutique selon l'invention se présente sous forme de comprimés contenant un coeur incluant du vorapaxar, de l'atopaxar ou de la 3-2-(chloro-phényl)-1-[4-(4-fluoro-benzyl)-pipérazin-1-yl]-propénone, en mélange avec les excipients suivants : carbonate de sodium, glycine, povidone, cellulose microcristalline, silice, stéarate de calcium, dioxyde de titane, oxyde de fer.

Le coeur est enrobé de copolymère d'acide méthacrylique Eudragit^{®} L100 pour permettre une protection gastro-résistante de ce coeur, avec une libération à partir du duodénum.

Chaque comprimé contient une quantité comprise entre 1 et 10 mg de principe actif et entre 0,01 et 1,0 mg d'Eudragit ^{®} L100.

### Posologie

Pour chacune des formules ci-dessus, la composition pharmaceutique peut être administrée à raison d'1 comprimé par jour en 1 unique prise.

Le traitement débute de préférence au plus tôt dès le diagnostic de la maladie, et, de préférence, dans les 12 premiers mois suivant l'événement aigu.

### Exemple 4 : effet de réparation des cellules épithéliales

Sur des cellules épithéliales de côlon humain Caco2 en monocouche et à confluence, une lésion est pratiquée à l'aide d'un forceps stérile.

Les cellules sont ensuite rincées par 2 fois avec du sérum de veau foetal stérile puis placées dans un milieu de culture approprié. Dans ce milieu est ajouté, dans des boites de culture indépendantes, du vorapaxar à différentes concentrations (10 nM, 100 nM, 1 µM, 10 µM) ou le véhicule (témoin).

La cicatrisation de la lésion est suivie et analysée à l'aide d'un microscope confocal pendant 28 h. Le pourcentage de surface de lésion lésée est déterminé pour chaque condition testée, 4 h, 22 h et 28 h après la lésion. Les résultats obtenus sont montrés sur la figure 12, respectivement en a/, b/ et c/.

En a/ sur la figure, il apparait que 4 h après la lésion la cicatrisation est fortement augmentée par le vorapaxar aux concentrations de 100 nM, 1 µM et 10 µM. Après 22 h, en b/ sur la figure, on observe que le vorapaxar amplifie la surface de cicatrisation de façon concentration dépendante. Enfin, en c/ sur la figure, on observe qu'après 28 h, le vorapaxar à la concentration de 100 nm à 10 µM a provoqué la cicatrisation complète de la lésion épithéliale.

Cette propriété de cicatrisation participe très probablement à la réduction de l'épaisseur du colon inflammé dans le modèle du TNBS chez le rat, montré sur la figure 5.

Ce résultat démontre que le vorapaxar est capable, par une amplification du processus de cicatrisation, de réparer la paroi épithéliale d'un intestin lésée, telle qu'elle peut subvenir dans les cas d'hyperperméabilité qui précède la poussée inflammatoire chez les patients atteints de la maladie de Crohn ou de la rectocolite hémorragique présentant une récidive de douleur.

## Revendications

1. Antagoniste de PAR-1 choisi dans le groupe constitué du vorapaxar, de l'atopaxar, de la 3-2-(chloro-phényl)-1-[4-(4-fluoro-benzyl)-pipérazin-1-yl]-propénone et de leurs sels pharmaceutiquement acceptables, pour son utilisation pour réduire la douleur et/ou réparer les tissus épithéliaux de l'intestin chez un sujet atteint d'une maladie inflammatoire chronique de l'intestin et du côlon.

2. Antagoniste de PAR-1 selon la revendication 1, pour son utilisation pour réduire la douleur et/ou réparer les tissus épithéliaux de l'intestin chez un sujet atteint d'une maladie inflammatoire chronique de l'intestin et du côlon, selon laquelle ledit sujet est un mammifère, de préférence un être humain.

3. Antagoniste de PAR-1 selon l'une quelconque des revendications 1 à 2, pour son utilisation pour réduire la douleur et/ou réparer les tissus épithéliaux de l'intestin chez un sujet atteint de la maladie de Crohn.

4. Composition pharmaceutique contenant un antagoniste de PAR-1 en tant que principe actif et au moins un excipient pharmaceutiquement acceptable, ledit antagoniste de PAR-1 étant choisi dans le groupe constitué du vorapaxar, de l'atopaxar, de la 3-2-(chloro-phényl)-1-[4-(4-fluoro-benzyl)-pipérazin-1-yl]-propénone et de leurs sels pharmaceutiquement acceptables, pour son utilisation pour réduire la douleur et/ou réparer les tissus épithéliaux de l'intestin chez un sujet atteint d'une maladie inflammatoire chronique de l'intestin et du côlon .

5. Composition pharmaceutique selon la revendication 4, pour son utilisation pour réduire la douleur et/ou réparer les tissus épithéliaux de l'intestin chez un sujet atteint d'une maladie inflammatoire chronique de l'intestin et du côlon, selon laquelle ledit sujet est un mammifère, de préférence un être humain.

6. Composition pharmaceutique selon l'une quelconque des revendications 4 à 5, pour son utilisation pour réduire la douleur et/ou réparer les tissus épithéliaux de l'intestin chez un sujet atteint de la maladie de Crohn.

7. Composition pharmaceutique selon l'une quelconque des revendications 4 à 6, pour son utilisation pour réduire la douleur et/ou réparer les tissus épithéliaux de l'intestin chez un sujet atteint d'une maladie inflammatoire chronique de l'intestin et du côlon, ladite composition présentant une forme galénique adaptée à une administration par voie orale.

8. Composition pharmaceutique selon l'une quelconque des revendications 4 à 7, pour son utilisation pour réduire la douleur et/ou réparer les tissus épithéliaux de l'intestin chez un sujet atteint d'une maladie inflammatoire chronique de l'intestin et du côlon, dans laquelle ledit antagoniste de PAR-1 est inclus dans un coeur recouvert d'un enrobage gastro-résistant.

## Patentansprüche

1. PAR-1-Antagonist, ausgewählt aus der Gruppe, die aus Vorapaxar, Atopaxar, 3-2-(Chlor-phenyl)-1-[4-(4-fluorobenzyl)-piperazin-1-yl]-propenon und deren pharmazeutisch akzeptablen Salzen besteht, für dessen Verwendung zur Reduzierung des Schmerzes und/oder zur Reparatur der Epithelgewebe des Darms bei einem Individuum, das an einer chronischen entzündlichen Erkrankung des Darms und des Kolons leidet.

2. PAR-1-Antagonist nach Anspruch 1 für dessen Verwendung zur Reduzierung des Schmerzes und/oder zur Reparatur der Epithelgewebe des Darms bei einem Individuum, das an einer chronischen entzündlichen Erkrankung des Darms und des Kolons leidet, wobei das Individuum ein Säugetier, vorzugsweise ein Mensch ist.

3. PAR-1-Antagonist nach einem der Ansprüche 1 bis 2 für dessen Verwendung zur Reduzierung des Schmerzes und/oder zur Reparatur der Epithelgewebe des Darms bei einem Individuum, das an Morbus Crohn leidet.

4. Pharmazeutische Zusammensetzung, die einen PAR-1-Antagonisten als Wirkstoff und mindestens einen pharmazeutisch akzeptablen Hilfsstoff enthält, wobei der PAR-1-Antagonist aus der Gruppe ausgewählt ist, die aus Vorapaxar, Atopaxar, 3-2-(Chlor-phenyl)-1-[4-(4-fluorobenzyl)-piperazin-1-yl]-propenon und deren pharmazeutisch akzeptablen Salzen besteht, für deren Verwendung zur Reduzierung des Schmerzes und/oder zur Reparatur der Epithelgewebe des Darms bei einem Individuum, das an einer chronischen entzündlichen Erkrankung des Darms und des Kolons leidet.

5. Pharmazeutische Zusammensetzung nach Anspruch 4 für deren Verwendung zur Reduzierung des Schmerzes und/oder zur Reparatur der Epithelgewebe des Darms bei einem Individuum, das an einer chronischen entzündlichen Erkrankung des Darms und des Kolons leidet, wobei das Individuum ein Säugetier, vorzugsweise ein Mensch ist.

6. Pharmazeutische Zusammensetzung nach einem der Ansprüche 4 bis 5 für deren Verwendung zur Reduzierung des Schmerzes und/oder zur Reparatur der Epithelgewebe des Darms bei einem Individuum, das an Morbus Crohn leidet.

7. Pharmazeutische Zusammensetzung nach einem der Ansprüche 4 bis 6 für deren Verwendung zur Reduzierung des Schmerzes und/oder zur Reparatur der Epithelgewebe des Darms bei einem Individuum, das an einer chronischen entzündlichen Erkrankung des Darms und des Kolons leidet, wobei die Zusammensetzung eine galenische Form aufweist, die für eine Verabreichung auf oralem Weg geeignet ist.

8. Pharmazeutische Zusammensetzung nach einem der Ansprüche 4 bis 7 für deren Verwendung zur Reduzierung des Schmerzes und/oder zur Reparatur der Epithelgewebe des Darms bei einem Individuum, das an einer chronischen entzündlichen Erkrankung des Darms und des Kolons leidet, wobei der PAR-1-Antagonist in einem Kern enthalten ist, der von einer enterischen Umhüllung bedeckt ist.

## Claims

1. PAR-1 antagonist selected from the group constituted by vorapaxar, atopaxar, 3-2-(chloro-phenyl)-1-[4-(4-fluoro-benzyl)-piperazine-1-yl]propenone and their pharmaceutically acceptable salts, for its use to reduce the pain and/or repair the epithelial tissues of the intestine in a subject suffering from a chronic inflammatory bowel disease.

2. PAR-1 antagonist according to claim 1, for its use to reduce the pain and/or repair the epithelial tissues of the intestine in a subject suffering from a chronic inflammatory bowel disease, wherein said subject is a mammal, preferably a human being.

3. PAR-1 antagonist according to any one of claims 1 to 2, for its use to reduce the pain and/or repair the epithelial tissues of the intestine in a subject suffering from Crohn's disease.

4. Pharmaceutical composition containing a PAR-1 antagonist as an active substance and at least one pharmaceutically acceptable excipient, said PAR-1 antagonist being selected from the group constituted by vorapaxar, atopaxar, 3-2-(chloro-phenyl)-1-[4-(4-fluoro-benzyl)-piperazine-1-yl]propenone and their pharmaceutically acceptable salts, for its use to reduce the pain and/or repair the epithelial tissues of the intestine in a subject suffering from a chronic inflammatory bowel disease.

5. Pharmaceutical composition according to claim 4, for its use to reduce the pain and/or repair the epithelial tissues of the intestine in a subject suffering from a chronic inflammatory bowel disease, wherein said subject is a mammal, preferably a human being.

6. Pharmaceutical composition according to any one of claims 4 to 5, for its use to reduce the pain and/or repair the epithelial tissues of the intestine in a subject suffering from Crohn's disease.

7. Pharmaceutical composition according to any one of claims 4 to 6, for its use to reduce the pain and/or repair the epithelial tissues of the intestine in a subject suffering from a chronic inflammatory bowel disease, said composition having a dosage form suited for an oral administration.

8. Pharmaceutical composition according to any one of claims 4 to 7, for its use to reduce the pain and/or repair the epithelial tissues of the intestine in a subject suffering from a chronic inflammatory bowel disease, wherein said PAR-1 antagonist is included in a core covered with an enteric coating.
